# EUROPEAN PATENT SPECIFICATION

(11) **EP 0 525 929 B1**
(45) Date of publication and mention of the grant of the patent: **21.12.1994**
(21) Application number: 92301783.4
(22) Date of filing: 02.03.1992
(51) Int. Cl.: H01F 1/00

(54) **Process for producing organic ferromagnetic sustances**
Verfahren zur Herstellung organischer ferromagnetischer Substanzen
Procédé pour la fabrication de substances organique ferromagnétiques

(30) Priority: 25.06.1991 JP 180213/91
(43) Date of publication of application: 03.02.1993
(73) Proprietor: DIRECTOR-GENERAL OF THE AGENCY OF INDUSTRIAL SCIENCE AND TECHNOLOGY, Tokyo (JP)
(72) Inventor: Murata, Kazuhisa, Tsukuba-shi, Ibaraki-ken (JP); Matsuda, Akio, Kashiwa-shi, Chiba-ken (JP); Masuda, Takashi, Abiko-shi, Chiba-ken (JP)
(74) Representative: Allam, Peter Clerk

(56) References cited:
- EP-A- 0 414 537
- PATENT ABSTRACTS OF JAPAN vol. 015, no. 072 (C-0808)20 February 1991
- CHEMICAL ABSTRACTS, vol. 116, no. 24, Columbus, Ohio, US; abstract no.246657Y, ARAKI ET AL.: 'FERROMAGNETIC AND FERRIMAGNETIC BEHAVIORS OF PYROLYSEDORGANIC MOLECULES CONTAINING NITROGEN ATOMS'
- CHEMICAL ABSTRACTS, vol. 116, no. 18, Columbus, Ohio, US; abstract no.186273H, ARAKI ET AL.: 'FERROMAGNETIC BEHAVIOR OF PYROLYSED ORGANIC MOLECULESCONTAINING NITROGEN ATOMS'

## Description

This invention relates to a process for producing an organic ferromagnetic substance.

As metal-free organic ferromagnetic substances, there are known (i) diacetylene having a nitroxy radical in the 1,4-position thereof, (ii) polymers synthesized from pyrene and benzaldehyde, (iii) triaminobenzene polymers and (iv) indigo polymers. These conventional organic ferromagnetic substances, however, have the defects that they have a low saturation magnetization (about 0.5 emu/g or less) and require many steps for their synthesis.

EP-A-0414537 discloses a process for producing an organic ferromagnetic substance, which comprises heating at least one polymer selected from (a) an aromatic polymer having a structure such that the aromatic compounds are connected via alkylene chains which may be substituted by an alkyl or aryl group, (b) a polymer of a triarylmethane, (c) a polymer of an acetylene compound and (d) a copolymer of an α,β-unsaturated compound, singly or together with an auxiliary inorganic material and/or material other than the above polymers (a)-(d), to a temperature above 500°C under vacuum or in the presence of an inert gas to form a carbonaceous substance in mid course of graphitization which contains 1-10 hydrogen atoms per 100 carbon atoms.

JP-A-2-296710 discloses a process for producing an organic ferromagnetic substance, in which an organic compound with a molecular weight of not more than 500, for example methane, benzene and adamantane, is deposited by gas-phase chemical vapor deposition at 500-1500°C in an inert atmosphere.

It is an object of the present invention to provide a process for producing an organic ferromagnetic substance which is easy to perform and which results in a ferromagnetic substance which has excellent stability and which shows a high saturation magnetization.

In accordance with the present invention there is provided a process for producing an organic ferromagnetic substance, which comprises heating at least one organic compound having a hydrogen-to-carbon ratio of 1.7:1 or more and selected from aza compounds, crown compounds and alkylamines to a temperature of 500°C or higher under vacuum or in an inert gas so as to form an amorphous carbonaceous substance in mid course of graphitization containing from 1-50 hydrogen atoms per 100 carbon atoms.

The organic compounds to be used in the present invention are those which have a hydrogen-to-carbon ratio of 1.7 or more, and which are aza compounds, crown compounds or alkylamines.

A first type of the organic compounds used in the present invention are aza compounds. Examples thereof are chained or cyclic tetraza compounds represented by the following general formulae (III) or (IV):

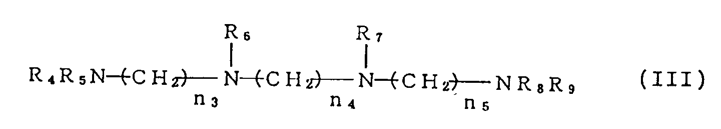

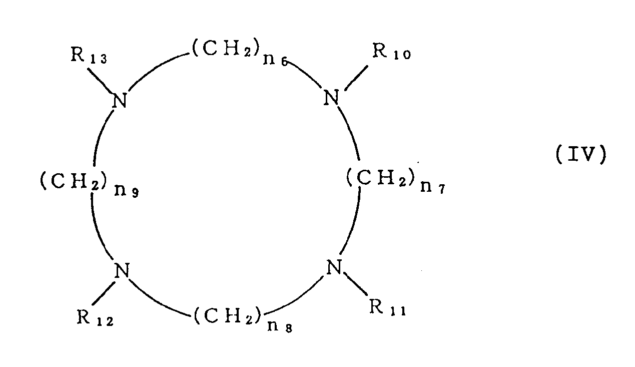

In the above general formulae, R₄ - R₁₄ are the same or different and each represents a hydrogen atom or an alkyl group, and n₃ - n₉ each represents an integer of 1 or more. Specific examples of the compounds represented by the general formula (III) include 1,4,8,11-tetrazaundecane, 1,5,8,12-tetrazadodecane and 1,5,9,13-tetrazatridecane. Specific examples of the compounds represented by the general formula (IV) include 1,4,8,11-tetrazacyclotetradecane and 1,4,8,12-tetrazacyclopentadecane.

A second type of useful organic compounds are crown compounds. Typical examples thereof are represented by the following general formulae (V) and (VI):

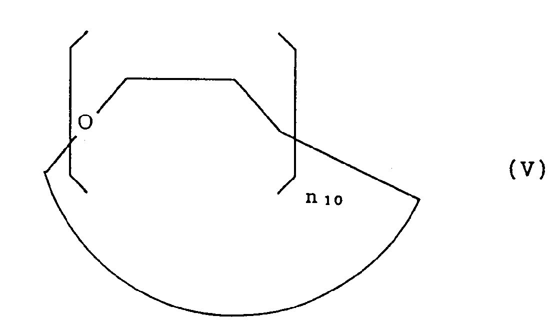

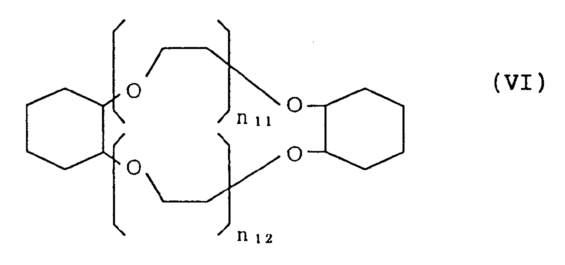

In the above general formulae, n₁₀ - n₁₂ each represents an integer of 1 or more, preferably 4 - 8. Specific examples of the compounds represented by the general formula (V) include 15-crown-5,18-crown-6, etc. Specific examples of the compounds represented by the general formula (VI) include cyclohexano-18-crown-6 and dicyclohexano-24-crown-8.

The third type of useful organic compounds are alkylamine compounds represented by the following general formula (VII): 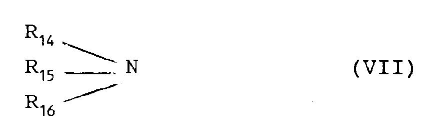
wherein R₁₄-R₁₆ are the same or different and each stands for a hydrogen atom, an alkyl group, an alkoxy group or a hydroxy group, at least one of R₁₄-R₁₆ being an alkyl group.

The process of the present invention is carried out by subjecting the compound or compounds having a hydrogen-to-carbon ratio of 1.7 or more to a heat treatment at a temperature above 500°C, preferably at a temperature of 900-1100°C, preferably for 0.1-10 hours, more preferably 0.3-2 hours, under vacuum or in the presence of an inert gas (e.g. an argon gas or a nitrogen gas). The upper limit of the heating temperature is about 3000°C.

In order to obtain a ferromagnetic amorphous carbonaceous substance by thermal carbonization of a specific organic compound according to the process of the present invention, it is necessary to control the heating conditions so that there results a carbonaceous substance in mid course of graphitization containing hydrogen atoms. The number of hydrogen atoms in the carbonaceous substance is 1-50 per 100 carbon atoms.

The presence of a carbonaceous substance in mid course of graphitization can be shown by X-ray diffraction and Laser-Raman analyses. In X-ray diffraction analysis, a diffraction line (002) showing absorption of carbon is usually detected in the vicinity of 2Θ = 25° with graphite, but is not clearly detected with a carbonaceous substance in mid course of graphitization. Laser-Raman analysis also does not show an absorption at 1580 cm⁻¹' which is specific to graphite when the carbonaceous substance is partially graphitized.

The present invention will now be illustrated in more detail by way of examples and comparative examples.

### Comparative Example 1

0.5 g of cyclododecane (hydrogen/carbon = 2) was placed in a quartz reaction tube at the left end thereof and, after outgassing the tube under vacuum for about 3 hours, the right end of the tube was heated to 950°C. After the heated end became a reaction temperature, cyclododecane at the left end was heated to evaporate. The evaporated cyclododecane was guided to the heated right end of the tube. After completion of the reaction at 950°C for 1 hour, the tube was cooled, and an amorphous carbonaceous product adhering to the reaction tube surface was taken out (about 0.04 g). At least part of the thus-obtained substance showed the property of being attracted by a permanent magnet (6000 gausses). This attracted portion (about 0.01 g) showed on the measurement of coercive force Hc and saturation magnetization Imax at ordinary temperature, an Hc of 8121 A/m (102 Oe) and an Imax of 1.52 x 10⁻⁶ Wb.m/kg (1.21 emu/g), respectively. Elementary analysis of the carbonaceous substance revealed that the substance had a hydrogen-to-carbon ratio (H/C) of 0.305. In the powder X-ray diffraction analysis, this carbonaceous substance did not show a clear diffraction line of carbon (002) in the vicinity of 2Θ = 25° which is observed with ordinary graphite, thus the carbonaceous substance being shown to be an amorphous substance.

### Comparative Examples 2-5 and Examples 1-7

The same reaction as in Comparative Example 1 was conducted except for changing the organic compound. Portions of the products attracted by a permanent magnet were measured as in Comparative Example 1 to obtain results as shown in Table 1.

It will be noted from Table 1 that the amorphous carbonaceous substances obtained when the starting organic compound was an aza compound, crown compound or alkylamine in accordance with the present invention (Examples 1-7) had higher, and in some cases significantly higher, Imax values than when other organic compounds were employed (Comparative Examples 1-5), even though these organic compounds had H/C ratios of greater than 1.7:1.

### Comparative Examples 6-7

The same reaction as in Comparative Example 1 was conducted except for changing cyclododecane by 0.5 g of pyrene (hydrogen/carbon = 0.625) or 0.5 g of tetrahydrocarbazole (hydrogen/carbon = 1.083). The thus-obtained carbonaceous substances showed very low saturation magnetization values as shown in Table 1.

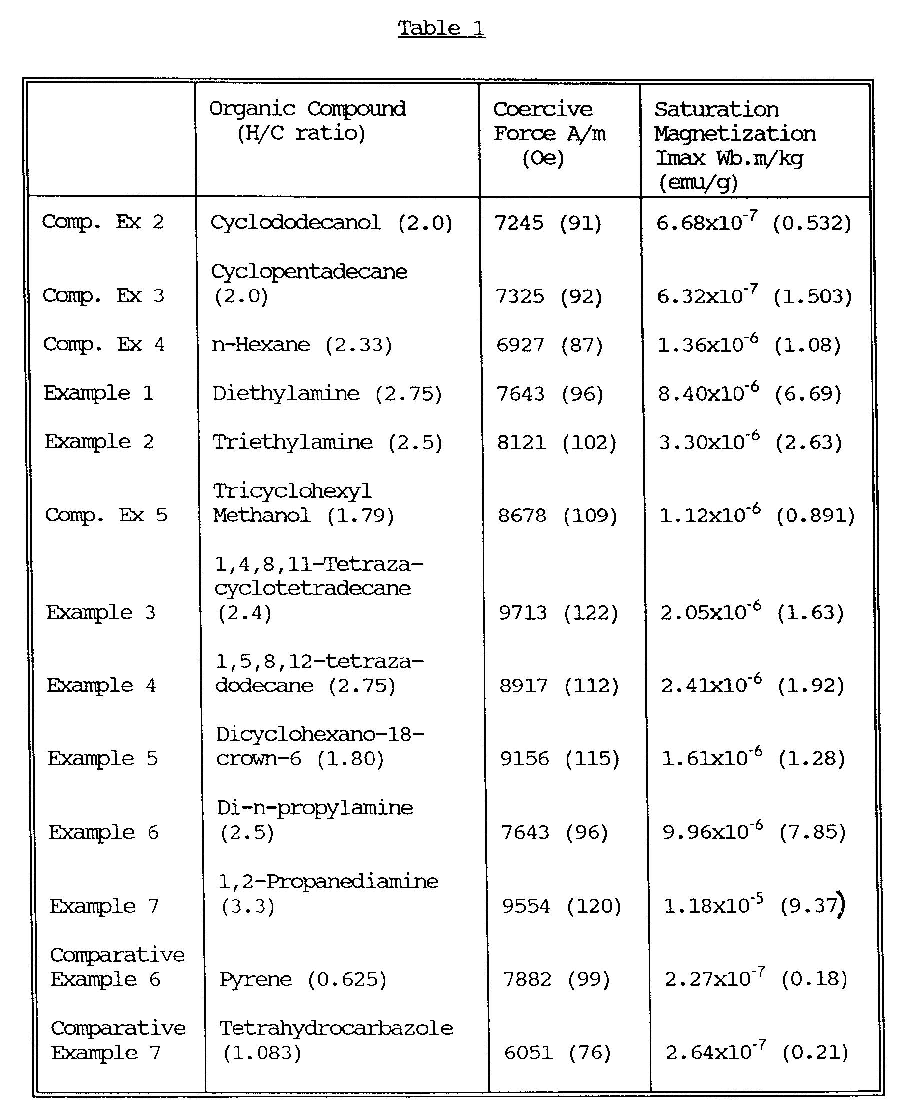

The organic ferromagnetic substance comprising the amorphous carbonaceous substance produced in accordance with the present invention from specific organic compounds is advantageously used as an industrial product such as a toner of copiers and can find wide applications utilizing its ferromagentic properties.

## Claims

1. A process for producing an organic ferromagnetic substance, which comprises heating at least one organic compound having a hydrogen-to-carbon ratio of 1.7:1 or more and selected from aza compounds, crown compounds and alkylamines to a temperature of 500°C or higher under vacuum or in an inert gas so as to form an amorphous carbonaceous substance in mid course of graphitization containing from 1-50 hydrogen atoms per 100 carbon atoms.

2. A process as set forth in Claim 1, wherein said aza compound is represented by the following general formulae (III) or (IV): 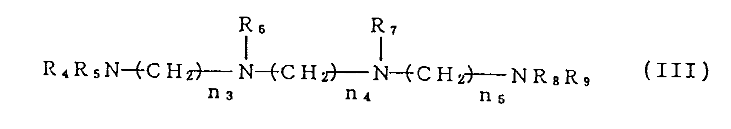 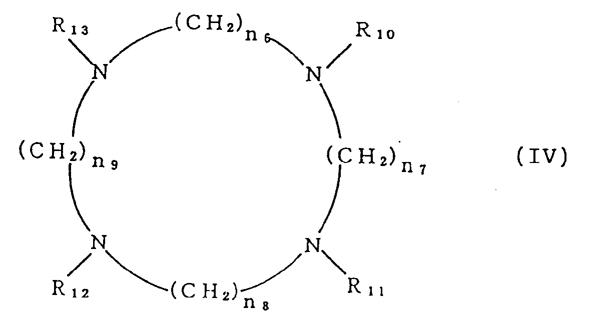 wherein R₄-R₁₄ are the same or different and each represents a hydrogen atom or an alkyl group, and n₃-n₉ each represents an integer of 1 or more.

3. A process as set forth in Claim 1, wherein said crown compound is represented by the following general formulae (V) or (VI): 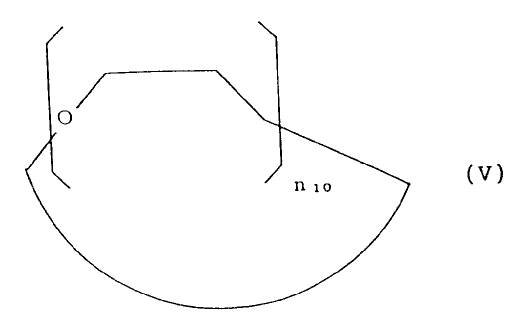 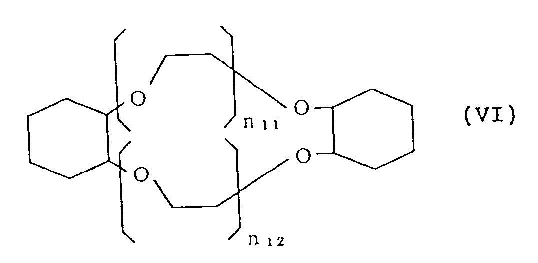 wherein n₁₀-n₁₂ each represents an integer of 1 or more.

4. A process as set forth in Claim 1, wherein said alkylamine is represented by the following general formula (VII): 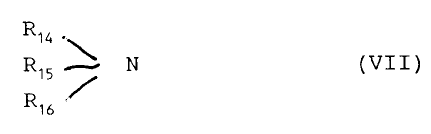 wherein R₁₄-R₁₆ are the same or different and each stands for a hydrogen atom, an alkyl group, an alkoxy group or a hydroxy group, with the proviso that at least one of R₁₄-R₁₆ is an alkyl group.

## Patentansprüche

1. Verfahren zur Herstellung einer organischen ferromagnetischen Substanz, das das Erhitzen von mindestens einer organischen Verbindung mit einem Wasserstoff-Kohlenstoff-Verhältnis von mindestens 1,7:1, die unterAzaverbindungen, Kronenverbindungen und Alkylaminen ausgewählt wird, unter Vakuumbedingungen oder in einer Inertgasatmosphäre auf eine Temperatur von mindestens 500 °C umfaßt, so daß im Verlauf der Graphitierung eine amorphe kohlenstoffhaltige Substanz gebildet wird, die 1 bis 50 Wasserstoffatome je 100 Kohlenstoffatome enthält.

2. Verfahren nach Anspruch 1, bei dem die Azaverbindung durch die folgenden allgemeinen Formeln (III) und (IV) wiedergegeben wird: 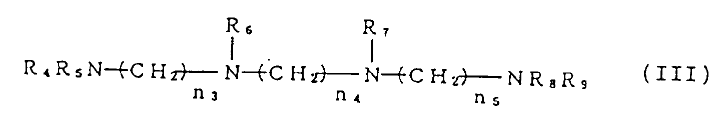 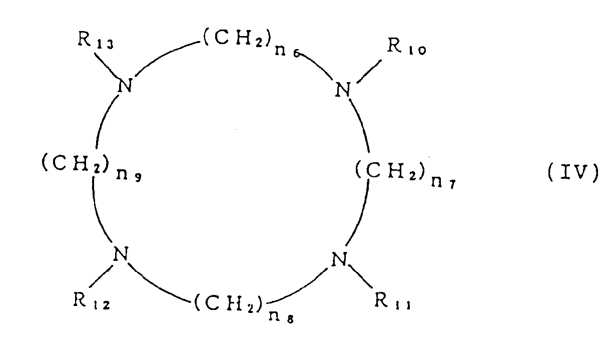 worin R₄ bis R₁₄ identisch oder unterschiedlich sind und jeweils ein Wasserstoffatom oder eine Alkylgruppe darstellen und n₃ bis n₉ jeweils eine ganze Zahl und mindestens 1 bedeuten.

3. Verfahren nach Anspruch 1, bei dem die Kronenetherverbindung durch die folgenden allgemeinen Formeln (V) und (VI) wiedergegeben wird: 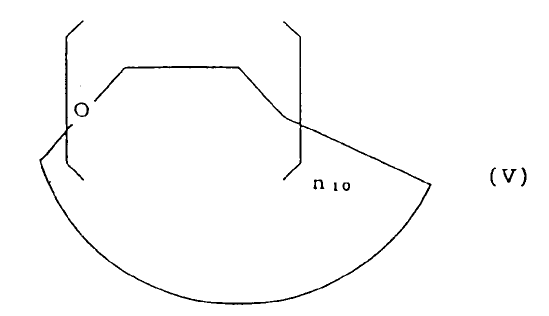 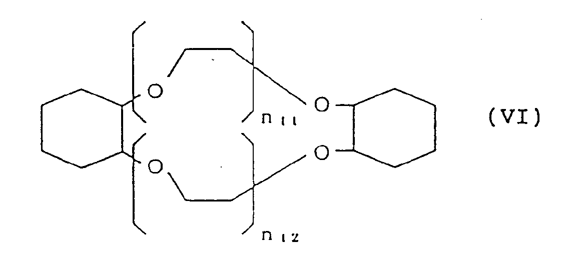 worin n₁₀ bis n₁₂ jeweils eine ganze Zahl und mindestens 1 bedeuten.

4. Verfahren nach Anspruch 1, bei dem das Alkylamin durch die folgende allgemeine Formel (VII) wiedergegeben wird: 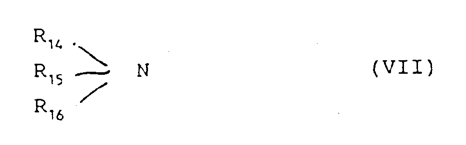 worin R₁₄ bis R₁₀ identisch oder unterschiedlich sind und jeweils für ein Wasserstoffatom, eine Alkylgruppe, eine Alkoxylgruppe oder eine Hydroxylgruppe stehen unter der Bedingung, daß es sich zumindest bei einem von R₁₄ bis R₁₆ um eine Alkylgruppe handelt.

## Revendications

1. Procédé de fabrication d'une substance ferromagnétique organique qui comprend le fait de chauffer au moins un composé organique ayant un rapport hydrogène/carbone de 1,7:1 ou plus et choisi parmi les composés aza, les composés en couronne et les alkylamines à une température de 500°C ou plus, sous vide ou sous gaz inerte, afin de former une substance carbonée amorphe à mi-chemin de la graphitisation et contenant de 1 à 50 atomes d'hydrogène pour 100 atomes de carbone.

2. Procédé selon la revendication 1, dans lequel ledit composé aza est représenté par les formules générales (III) ou (IV) suivantes : 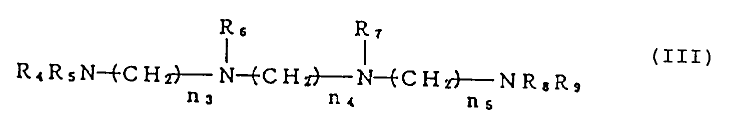 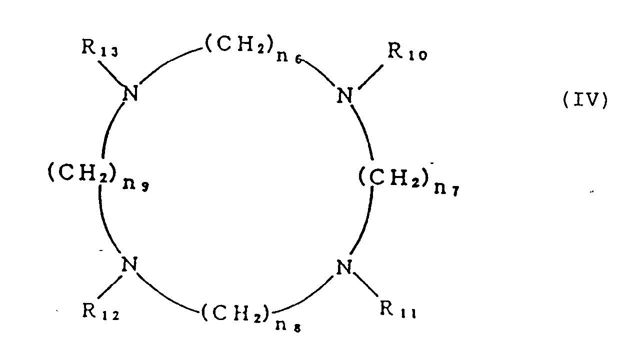 dans lesquelles R₄-R₁₄ sont identiques ou différents et représentent chacun un atome d'hydrogène ou un groupement alkyle, et n₃-n₉ représentent chacun un nombre entier supérieur ou égal à 1.

3. Procédé selon la revendication 1, dans lequel ledit composé à couronne est représenté par les formules générales (V) et (VI) suivantes: 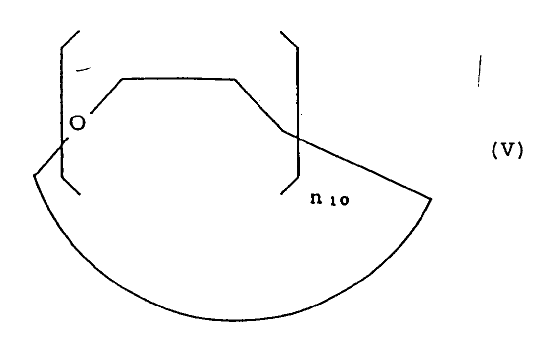 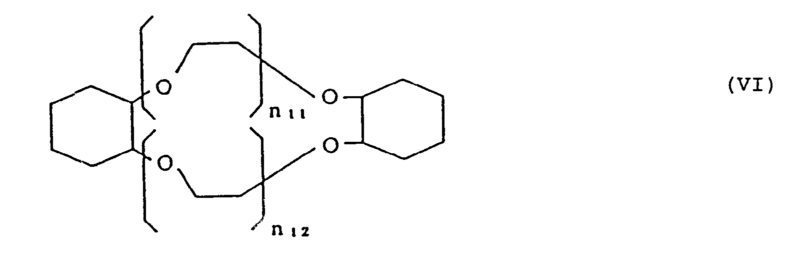 dans lesquelles n₁₀-n₁₂ représentent chacun un entier supérieur ou égal à 1.

4. Procédé selon la revendication 1, dans lequel ledit alkylamine est réprésenté par la formule générale (VII) suivante : 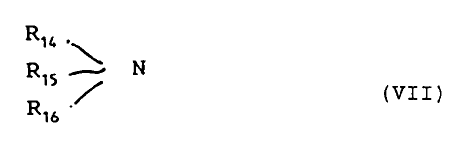 dans laquelle R₁₄-R₁₆ sont identiques ou différents et sont chacun un atome d'hydrogène, un groupement alkyle, un groupement alcoxy ou un groupement hydroxy, au moins l'un de R₁₄-R₁₆ étant un groupement alkyle.
